**Europäisches Patentamt**

(19) **European Patent Office**

**Office européen des brevets**

(11) Veröffentlichungsnummer: **0 114 048 B1**

(12) # EUROPÄISCHE PATENTSCHRIFT

(45) Veröffentlichungstag der Patentschrift:
28.01.87

(51) Int. Cl.⁴: **A 61 K 31/40** // C07D209/88

(21) Anmeldenummer: **84100098.7**

(22) Anmeldetag: **07.01.84**

(54) Verwendung von D,L- und D-Carazolol zur Herstellung von Antiglaukommitteln sowie Arzneimittel, die D-Carazolol enthalten.

(30) Priorität: **13.01.83 DE 3300933**

(43) Veröffentlichungstag der Anmeldung:
**25.07.84 Patentblatt 84/30**

(45) Bekanntmachung des Hinweises auf die Patenterteilung:
**28.01.87 Patentblatt 87/5**

(84) Benannte Vertragsstaaten:
**AT BE CH DE FR GB IT LI LU NL SE**

(73) Patentinhaber: **Boehringer Mannheim GmbH, Sandhofer Strasse 116, D-6800 Mannheim 31 (DE)**

(72) Erfinder: **Bartsch, Wolfgang, Dr. med.vet., D-6806 Viernheim, Franconviller-Strasse 5 (DE)**

(56) Entgegenhaltungen:
DE - A - 2 240 599

ARZNEIMITTELFORSCHUNG, Band 27, Nr. 5, 1977, Seiten 1022-1026, Aulendorf, DE. W. BARTSCH et al.: "Cardiac action of Carazolol and methylpyranol in comparison with other beta-receptor blockers"
CHEMICAL ABSTRACTS, Band 95, Nr. 17, 26. Oktober 1981, Seite 9, Nr. 143760v, Columbus, Ohio, USA. E. BUERGISSER et al.: "Alternative explanation for the apparent "two-step" binding kinetics of high-affinity racemic antagonist radioliganda"
ARZNEIMITTELFORSCHUNG, Band 31(II), Nr. 11, 1981, Seiten 1885-1888, Aulendorf, DE. W. BARTSCH et al.: "Comparative investigation on the cardio-protective action of the beta-blockers carazolol, propranolol and pindolol in rats"

(56) Entgegenhaltungen: (Fortsetzung)
CHEMICAL ABSTRACTS, Band 96, Nr. 5, 1. Februar 1982, Seite 69, Nr. 28640Q, Columbus, Ohio, USA. H. J. MERTE: "Intraocular pressure and beta-receptor blockers"
CHEMICAL ABSTRACTS, Band 87, Nr. 11, 12. September 1977, Seite 52, Nr. 78551w, Columbus, Ohio, USA. T. J. ZIMMERMAN et al.: "Timolol. A beta-adrenergic blocking agent for the treatment of glaucoma"
CHEMICAL ABSTRACTS, Band 87, Nr. 11, 12. September 1977, Seite 52, Nr. 78552x, Columbus, Ohio, USA. T. J. ZIMMERMAN et al.: "Thimolol. Dose response and duration of action"

## Beschreibung

Carazolol ist der internationale Freiname für das racemische (D,L)-1-Carbazolyl-(4)-oxy-3-isopropylamin-propanol-(2). Seine ausgeprägte β-blockierende Wirkung an Mensch und Tier ist dokumentiert. Erstmals beschrieben wurde diese Verbindung in Beispiel 1 der DE-PS 22 40 599, wobei auch allgemein angegeben ist, dass der Wirkstoff als Injektionslösung appliziert werden kann. In einer Vielzahl weiterer Publikationen wurde die β-blockierende Wirkung in Vergleich zu bekannten β-Blockern herausgestellt. (W. Bartsch et al, Arzneim.- Forsch. 27 (I), 5, 1022 ff, 1977; W. Bartsch Excerpta medica, Amsterdam 1980, S. 44 ff.). Bekannt ist D,L-Carazolol unter der Bezeichnung Suarcron® als Betarezeptorenblocker für die Veterinärmedizin und unter der Bezeichnung Conducton ® in der Humanmedizin.

Es wurde nun überraschenderweise gefunden, dass D,L-Carazolol auch in der Augenheilkunde in extrem geringer Konzentration Verwendung finden kann und zwar zur Senkung des Augeninnendruckes bei der Glaukomtherapie.

Es ist bereits seit langer Zeit bekannt, dass β-Rezeptorenblocker wie Propranolol u.a. bei Glaukompatienten den Augendruck senken (vgl. G.K. Krieglstein, klin. Monatsblätter für Augenheilkunde 172, 677 ff. (1978)); jedoch zeigten sich bei der lokalen Anwendung von β-Blockern unangenehme Nebenwirkungen, die zu brennenden und stechenden Schmerzen am Auge führten, sowie eine Oberflächenanästhesie am Auge bewirkten. Deshalb sind β-Blocker dieses Typs für die Glaukomtherapie weniger geeignet.

Auch Timolol, das S-(-)1-(tert.Butylamino)-3-[(4-morpholino-1,2,5-thiadiazol-3-yl)oxy]-2-propanol, welches bei der Glaukomtherapie verwendet wird, zeigt in der handelsüblichen Form (0,5%ige Lösung), insbesondere in der Langzeittherapie Nebenwirkungen, wie signifikante Senkung des Blutdrucks und Entwicklung trockener Augen. (N.V. Nielsen, Z. prakt. Augenheilkunde 2: 71–77 (1981)).

Versuche mit D,L-Carazolol ergaben nun, dass eine signifikante Senkung des Augeninnendruckes bereits mit einer 0,01%igen Lösung von D,L-Carazolol erreicht wird, ohne dass bei dieser Dosierung trotz der hohen therapeutischen Wirksamkeit irgendwelche unerwünschten Nebenwirkungen beobachtet wurden.

Gegenstand der Erfindung ist somit die Verwendung von racemischen D,L-Carazolol zur Herstellung von Arzneimitteln für die Glaukombehandlung sowie Arzneimittel, die D-Carazolol enthalten, zur topischen Anwendung am Auge.

Experimentell ist gesichert, dass generell eine β-blockierende Qualität an die L-Isomere-Form gebunden ist (A.M. Barrett et al Brit. J., Pharmac., 34, (43–55)) 1968); dies gilt auch für Carazolol. Als seltene, aber sehr schwerwiegende Zwischenfälle sind nach Gabe von Timolol (L-Isomere-Form) in Folge der β-Blockade Asthma-Anfälle, Herz-Rhythmusstörungen und sogar einige Todesfälle in der Literatur beschrieben (A. Vonwil et al, Schweiz. med. Wschr. 111, 665–669, 1981).

Es wurde nun gefunden, dass bei D-Carazolol auch eine therapeutische Wirkung, nämlich eine signifikante Senkung des Augeninnendruckes, vorhanden ist, ohne dass β-Blocker-typische, d.h. systemische Nebenwirkungen auftreten.

Weiterer Gegenstand der Erfindung ist somit auch D-Carazolol zur Verwendung als Antiglaukom-Mittel, Arzneimittel, die D-Carazolol praktisch frei von L-Carazolol enthalten, zur topischen Anwendung am Auge und die Verwendung von D-Carazolol zur Herstellung solcher Arzneimittel.

D-Carazolol – eine andere Bezeichnung ist R(+)-Carazolol – wurde von S. Manulan et al. auf seine β-blockierende Wirkung untersucht [Circ.Res. 49, S. 326–336, (1981)], wobei keine diesbezügliche Wirkung festgestellt wurde.

Zur Glaukombehandlung werden D,L- und D-Carazolol bzw. ihre pharmakologisch unbedenklichen Salze in Form von Augentropfen verwendet. Bevorzugt sind Salze mit physiologisch verträglichen anorganischen oder organischen Säuren, z.B. Salzsäure, Bromwasserstoffsäure, Phosphorsäure, Schwefelsäure, Essigsäure, Salicylsäure, Citronensäure, Benzoesäure, Naphthoesäure, o-Acetoxybenzoesäure, Adipinsäure oder Maleinsäure.

Geeignet sind isotone Lösungen mit einem pH von ca. 7,0. Als Medium kommt vorzugsweise Wasser zur Anwendung, welches übliche Zusätze wie Konservierungsmittel, Lösungsvermittler oder Puffer enthalten kann. Als Konservierungsstoffe kommen vorzugsweise Benzylalkohol, Benzalkoniumchlorid, Phenol oder Chlorhexidinacetat in Frage. Lösungsvermittler sind insbesondere Polyethylenglykole, Polyvinylpyrrolidon oder Glycerin. Als Puffer werden bevorzugt Essigsäure/Natriumacetat, Citronensäure/Natriumcitrat oder Natrium-EDTA verwendet.

Untersuchungen am Menschen haben gezeigt, dass das racemische D,L-Carazolol bzw. seine Salze bereits in einer Konzentration von 0,005–0,03% bei Glaukompatienten den Augeninnendruck bei guter Verträglichkeit signifikant senkt. Hierzu werden Patienten einmal pro Tag mit D,L-Carazolol-Augentropfen behandelt. Als eine bevorzugte Rezeptur erwies sich eine 0,01%ige Lösung, die keine der bekannten Nebenwirkungen wie anästhetisches Gefühl am Auge oder im Kopfbereich und starkes Brennen zeigte.

Bei der Verwendung von D-Carazolol bzw. seinen Salzen wurde eine entsprechende signifikante Senkung des Augeninnendruckes ermittelt, wobei ebenfalls keinerlei Nebenwirkungen zu beobachten waren. In Antiglaukom-Rezepturen wurde D-Carazolol in Konzentrationen von 0,001–0,1% angesetzt, bevorzugt 0,005–0,01%.

Die Dosis, ausgedrückt durch die Menge der pro Tag benötigten Augentropfen, hängt von der Konzentration der Lösungen ab. In der Regel sollte zweimal ein Tropfen einer 0,01%igen D,L-oder 0,005%igen D-Carazolol-Lösung pro Auge genügen, jedoch können eventuell auch 3–4 Tropfen einer niedriger konzentrierten Lösung pro Tag verwendet werden.

Die nachfolgenden Beispiele beschreiben die Herstellung des D-Carazolols als Hydroacetat sowie Augentropfen mit D,L- bzw. D-Carazolol als Wirkstoff.

Beispiel 1

R(+) 1-Carbazolyl-(4)-oxy-3-isopropylamino-propanol-(2) (D-Carazolol)

178,8 g (0,6 Mol) D,L-Carazolol, 62,1 g (0,3 Mol) L-N-Acetyl-phenylalanin (LAP) und 18 g (0,3 Mol) Eisessig werden in 4,5 l Wasser zum Sieden erhitzt, wobei eine weitgehend klare Lösung entsteht. Diese wird über ein Faltenfilter filtriert und ohne Aussenkühlung kaltgerührt.

Sobald sich die Lösung bei ca. 65 °C zu trüben beginnt, wird mit etwas LAP/D-Carazololsalz angeimpft. Man lässt auf Raumtemperatur abkühlen und saugt das erhaltene Produkt ab.

Das feuchte Produkt wird in 2 250 ml Wasser heiss gelöst, über ein Faltenfilter filtriert, erneut auf Raumtemperatur kaltgerührt und das ausgefallene Produkt abgesaugt. Nach nochmaligem Umkristallisieren aus 1 800 ml bzw. 600 ml Wasser wird das feuchte Produkt (172 g) in 1,5 l Essigester und 1,5 l NaOH 1n geschüttelt, bis zwei klare Phasen entstanden sind. Nach der Phasentrennung wird die Essigesterphase noch dreimal mit je 450 ml Wasser ausgeschüttelt, über $Na_2SO_4$ getrocknet und i.Vak. bei ca. 50 °C Badtemperatur eingeengt.

Der Rückstand (43,2 g) wird aus 330 ml siedendem Toluol unter Zusatz einer Spur Aktivkohle umkristallisiert. Man lässt ohne Aussenkühlung auf Raumtemperatur kühlen, rührt dann 1–2 Stunden im Eisbad, saugt ab und trocknet bei 60 °C Umluft.

Das erhaltene Produkt wird unter Erwärmen auf ca. 65 °C in einem Gemisch aus 600 ml Toluol und 11 ml n-Butanol gelöst und über Nacht auf Raumtemperatur kaltgerührt. Man saugt ab und wäscht mit wenig Toluol nach. Dieser Vorgang wird mehrfach wiederholt. Anschliessend wird das Produkt in heissem Essigester gelöst und mit Eisessig versetzt. Das erhaltene Kristallisat wird nochmals mit Essigester unter Zusatz von Methanol umkristallisiert. Man erhält R(+) 1-Carbazolyl-(4)-oxy-3-isopropylamino-propanol-(2) (D-Carazolol) als Hydroacetat.

Fp: 158–160 °C $[\alpha]_D^{20}$ = −18,8° (C=1; Methanol) optische Reinheit nach GC-Befund: 99,5%.

Beispiel 2

Eine 0,01%ige Lösung von D,L-Carazolol zur topischen Glaukombehandlung wird hergestellt aus:

| | | |
|---|---:|---|
| Chlorhexidinacetat | 0,10 | mg |
| Natriumacetat x $3H_2O$ | 18,18 | mg |
| Essigsäure | 1 | mg |
| D,L-Carazolol | 0,1 | mg |
| Aqua dest. ad | 1000 | mg |

In der gleichen Weise erhält man 0,03 bzw. 0,005%ige Lösungen, von D,L-Carazolol, wenn man 0,3 bzw. 0,05 mg D,L-Carazolol einsetzt.

Beispiel 3

Eine 0,1, 0,01, 0,005 und 0,001%ige Lösung von D-Carazolol erhält man analog Beispiel 2, wenn man 1,0, 0,1, 0,05 oder 0,01 mg D-Carazolol als Wirkstoff einsetzt.

**Patentansprüche**

1. Verwendung von racemischem D,L-Carazolol und seiner pharmakologisch unbedenklichen Säureadditionssalze zur Herstellung von Arzneimitteln für die Glaukombehandlung.

2. D-Carazolol und seine pharmakologisch unbedenklichen Salze zur Verwendung bei der Glaukombehandlung.

3. Verwendung von D-Carazolol und seiner pharmakologisch unbedenklichen Säureadditionssalze zur Herstellung von Arzneimitteln für die Glaukombehandlung.

4. Wässrige Augentropfen, enthaltend D-Carazolol bzw. dessen Säureadditionssalze und die üblichen Puffer und Konservierungsstoffe.

5. Wässrige Augentropfen, enthaltend D,L-Carazolol als 0,005–0,03%ige Lösung bzw. dessen Säureadditionssalze und die üblichen Puffer-und Konservierungsstoffe.

6. Augentropfen gemäss Anspruch 4, mit D-Carazolol als 0,001–0,1%ige Lösung.

7. Verwendung von D,L- und D-Carazolol und ihrer pharmakologisch unbedenklichen Salze zur Herstellung von Augentropfen zur Glaukombehandlung.

**Revendications**

1. Utilisation de D,L-Carazolol racémique et de ses sels d'addition acide qui ne posent pas de problèmes d'ordre pharmacologique pour la fabrication de médicaments destinés au traitement du glaucome.

2. D-Carazolol et ses sels qui ne posent pas de problèmes d'ordre pharmacologique en vue de leur utilisation pour le traitement du glaucome.

3. Utilisation du D-Carazolol et de ses sels d'addition acide qui ne posent pas de problèmes d'ordre pharmacologique pour la fabrication de médicaments destinés au traitement du glaucome.

4. Gouttes de collyre aqueux contenant du D-Carazolol ou ses sels d'addition acide et les tampons et produits de conservation usuels.

5. Gouttes de collyre aqueux contenant du D-Carazolol sous la forme d'une solution à 0,005 à 0,03% ou ses sels d'addition acide et les tampons et produits de conservation usuels.

6. Gouttes de collyre selon la revendication 4, contenant du D-Carazolol sous forme de solution à 0,001 à 0,1%.

7. Utilisation de D,L- et de D-Carazolol et de leurs sels qui ne posent pas de problèmes d'ordre pharmacologique pour la fabrication de collyre destiné au traitement du glaucome.

**Claims**

1. The use of racemic D,L-carazolol and of its pharmacologically acceptable acid-addition salts for the preparation of medicaments for the treatment of glaucoma.

2. D-Carazolol and its pharmacologically acceptable salts for use in the treatment of glaucoma.

3. The use of D-carazolol and of its pharmacologically acceptable acid-addition salts for the preparation of medicaments for the treatment of glaucoma.

4. Aqueous eye drops, containing D-carazolol or its acid-addition salts and the conventional buffers and preserving agents.

5. Eye drops containing D,L-carazolol as 0.005–0.03% solution or its acid-addition salts and the conventional buffer and preserving agents.

6. Eye drops according to claim 4, with D-carazolol as 0.001–0.1% solution.

7. The use of D,L- and D-carazolol and of their pharmacologically acceptable salts for the preparation of eye drops for the treatment of glaucoma.